# EUROPEAN PATENT SPECIFICATION

(11) **EP 1 519 930 B1**
(45) Date of publication and mention of the grant of the patent: **20.10.2010**
(21) Application number: 03762807.0
(22) Date of filing: 08.07.2003
(51) Int. Cl.: C07D 307/93

(54) **BENZOPROSTACYCLIN INTERMEDIATES AND METHODS FOR THEIR PREPARATION**
BENZOPROSTACYCLINZWISCHENPRODUKTE UND VERFAHREN ZU DEREN HERSTELLUNG
INTERMEDIAIRES DE BENZOPROSTACYCLINE ET PROCEDES DE PREPARATION DE CES INTERMEDIAIRES

(30) Priority: 08.07.2002 GB 0215757
(43) Date of publication of application: 06.04.2005
(73) Proprietor: JOHNSON MATTHEY PUBLIC LIMITED COMPANY, 40-42 Hatton Garden London EC1N 8EE (GB)
(72) Inventor: CLISSOLD, Derek, Wyndham, Wokingham RG40 5YE (GB); MATHARU, Saroop, Singh, Cricklade, Swingdon SN6 6EB (GB)
(74) Representative: Swift, Jane Elizabeth Berrisford
(86) International application number: PCT/GB2003/002924
(87) International publication number: WO 2004/005274

(56) References cited:
- EP-A- 0 024 943
- EP-A- 0 084 856
- WO-A-03/011849

## Description

This invention relates to benzoprostacyclin intermediates, methods for their preparation, and products derived therefrom.

Since the discovery of prostacyclin (PGI₂), a number of chemically and metabolically stable PGI₂ analogues have been developed as clinically effective antithrombotic agents. See, for example, P.W. Collins and S.W. Djuric (Chem. Rev., 1993, 93, 1533); R. Nickolson, M.H. Town and H. Vorbrueggen (Med. Res. Rev., 1985, 5); and P.A. Aristof (Adv. Prostaglandin, Thromboxane, Leukotriene Res., 1985, 14, 309).

Among these, the benzoprostacyclins and in this class particularly the compound known as Beraprost (Figure 1), developed by Toray Industries Inc., is one of the better known compounds (see, for example, R.Kato, Y.Uji, K.Ono, K.Matsumoto, K.Hisano, I.Sakai, Y. Kagawa and M.Isai, (Jpn. J. Clin. Pharmacol. Ther., 1986, 17 , 267); R.Kato, Y.Uji, and K.Matsumoto, (Jpn. J. Clin. Pharmacol. Ther., 1989, 20 529); Isaka, N.Handa, M.Imaizumi, K.Kimura and T.Kamada (Thromb. Haemost., (1991, 65, 344)) and is a particularly valuable and established drug for the treatment of pulmonary vascular and peripheral vascular diseases.

The invention may however be used in relation to the synthesis of a variety of benzoprostacyclin compounds.

### Beraprost sodium (4 diastereoisomer mixture)

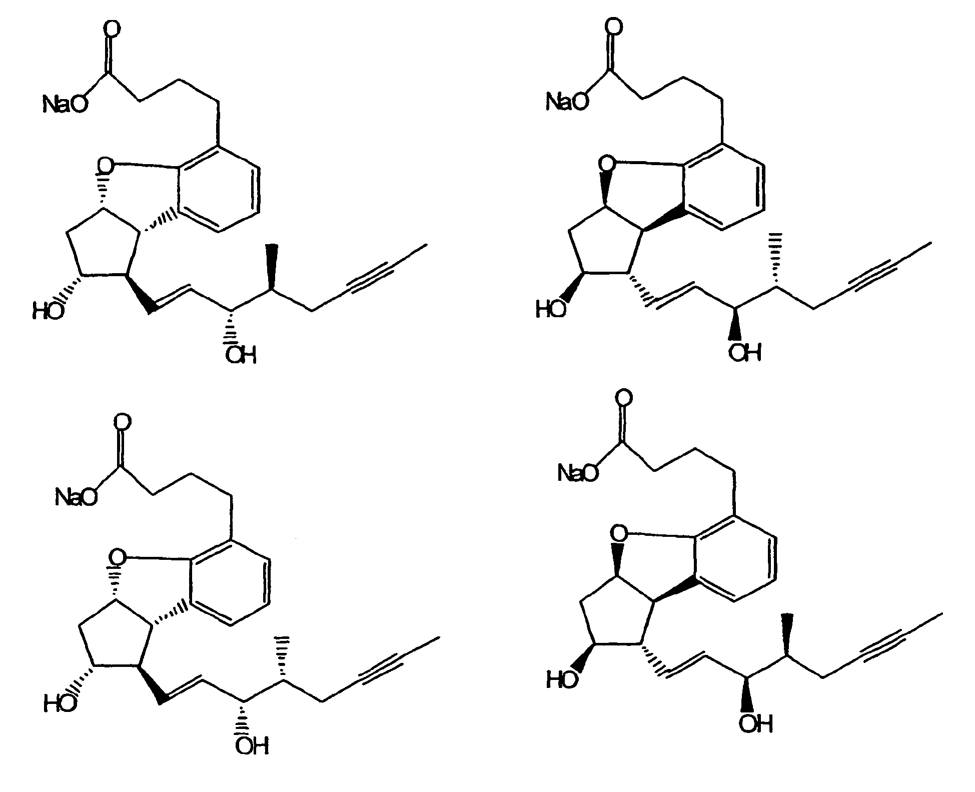

Beraprost and related benzoprostacyclins are complex molecules and available synthetic approaches leading to them are linear and rather inefficient.

This invention relates to the synthesis of Beraprost and related racemic benzoprostacyclin molecules via a convergent seven step approach based on the known racemic cyclopentenone intermediate (±)-2-(2-Allyloxy-3-bromophenyl)-4-hydroxy-cyclopent-2-enone shown as (1) in the reaction scheme below. See Y.Yushido, Y.Sato, S.Okamoto and F.Sato (J.Chem.Soc., Chem. Comm. 1995, 811, and JP-95/238046-A (Sato), for preparation of the starting material.

The invention has many aspects. In particularly preferred aspects, the invention is summarized in the appended claims. In broader aspects, the invention embraces equivalents thereof, including variations in reaction conditions, variation in reagents, and variation in the details of the appended and substituent groups and radicals in the molecules specifically described, while remaining within the general concept exemplified by the specific sequential synthesis shown in the inventive scheme set out below.

Referring to the scheme, in Step 1, the 4-hydroxy group is protected. This step may be carried out by conversion to any suitable protecting group, but preferably a *tert*-butyldimethylsilanyloxo group by reaction with *tert*-butyldimethylsilyl chloride. Suitable alternatives will be apparent to the skilled man.

In Step 2 the invention utilises the introduction of a latent aldehyde function, as an alkenyl side chain, into the appropriately protected cyclopentenone molecule (2). The synthetic sequence advantageously proceeds via a copper mediated 1,4 (Michael) addition of the appropriate alkenyl Grignard reagent to give a compound such as, preferably, (±)-2-(2-allyloxy-3-bromophenyl)-4-(*tert*-butyldimethylsilanyloxo)-3-(2-methylpropenyl)-cyclopentanone (3).

The preferred alkenyl side chain (2-methylpropenyl) offers particular advantages in this respect since the appropriate Grignard reagent, 2-methyl-1-propenyl magnesium bromide, is available commercially, reacts to give a double bond free from E, Z complications (thereby simplifying analysis and purification and avoiding the need to progress isomeric mixtures through several chemical steps), and gives an innocuous byproduct (acetone) on subsequent cleavage at Step 7.

Conceptually the introduction of the complete beta side chain would be an attractive and more convergent approach. However, the complexity of the side chain (two chiral centres) precludes this approach since stereochemical analysis demonstrates that eight isomers would result, in equal proportions, four of which are unwanted and thus presenting a severe and sacrificial separation/purification problem. The invention provides a route around this problem.

We refer to the introduction of alkenyl side chain with the object of subsequently converting it to an aldehyde as the introduction of a latent aldehyde function. A major advantage of this 'masked aldehyde' approach as applied to synthesis of Beraprost is that the latter is in fact a double racemate and thus consists of 4 stereoisomers. Introduction of an aldehyde function as detailed in the scheme sets up the correct stereochemistry in the molecule to enable introduction of the beta side chain via known (see for example EP-0 084 856-A1) Homer Emmons phosphonate chemistry. Beraprost is then accessible by conventional published methods.

A further feature of the invention relates to the efficient conversion of (3) to (4) in Step 3 of the scheme by a novel Palladium catalysed π-allyl reduction which has been discovered to enjoy a high degree of diastereoselectivity to generate the required R alcohol with simultaneous removal of the allyl protecting group. This allows the use of a simple borohydride reducing agent to generate at least a 10:1 ratio of R:S alcohols.

Since the next step in the procedure a Mitsunobu reaction (Step 4), will cyclise only the R alcohol, leaving any S alcohol unchanged, and the polarities of S-(4) and (5) are very different it is unnecessary to rigorously purify the product from Step 3. Purification of Step 4 product is readily and efficiently achieved.

Subsequent transformations to (8) by Steps 5, 6 and 7 may be accomplished, in high yield, by known means, preferably by a Suzuki coupling (Step 5), by osmium catalysed dihydroxylation (Step 6) and by periodate diol cleavage (Step 7). The conversion of (6) to (8) may, of course, by achieved by ozonolysis but the methods described herein offer advantages with respect to product purification.

This invention thereby provides an efficient (36% overall) method for generating the valuable and versatile intermediate (8) and allows the more economic production of Beraprost and related compounds.

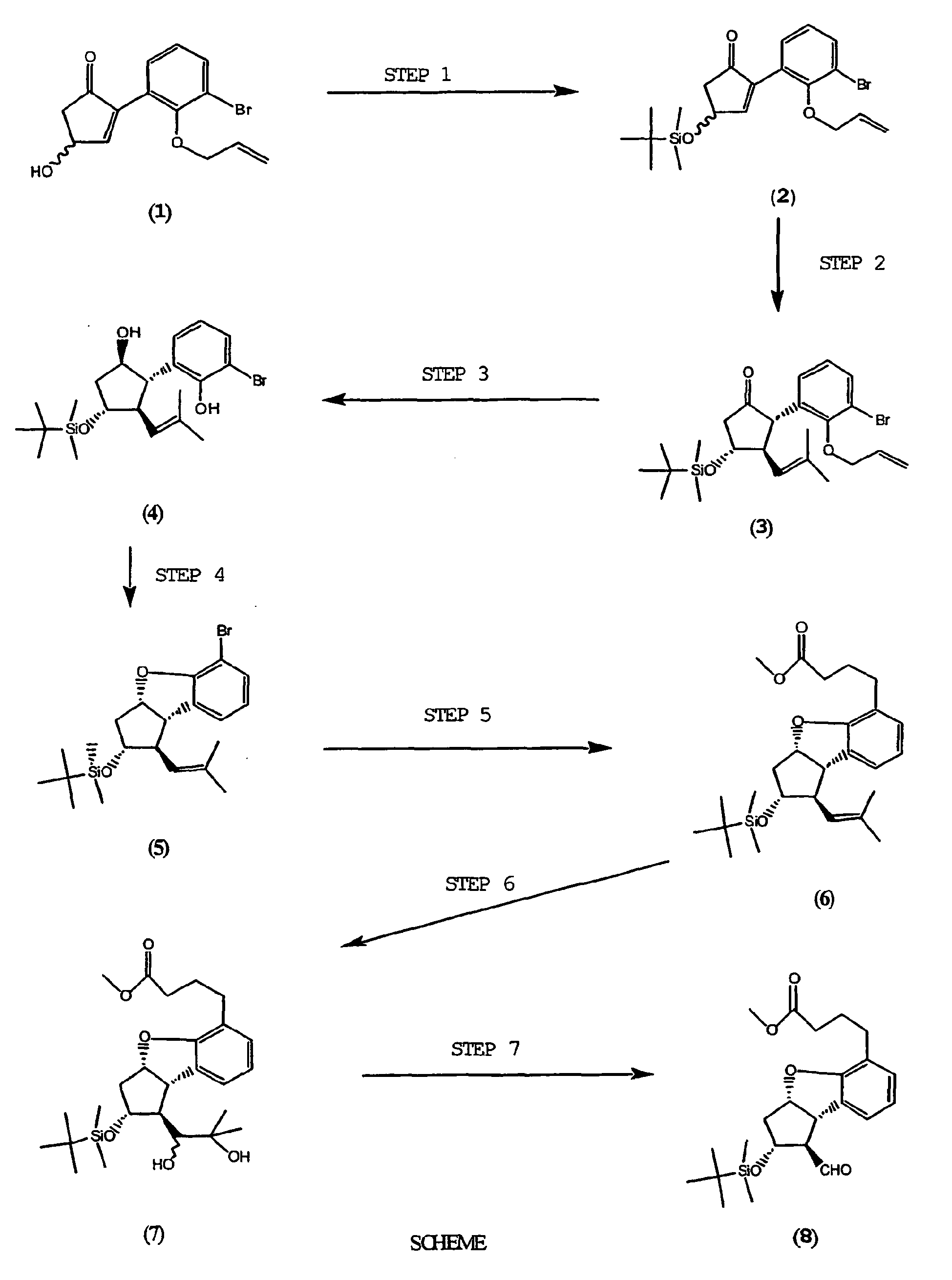

Various embodiments of the invention will be further illustrated in the following non-limiting specific Examples.

### Example 1 (Reaction scheme Step 1)

### (±)-2-(2-Allyloxy-3-bromophenyl)-4-(tert-butyldimethylsilanyloxo)-cyclopent-2-enone (2).

A solution of (1) (1084.2g, 3.51 mole, 1 equivalent) in dichloromethane (8.51) was prepared in a dry flask and charged with imidazole (286.3g, 4.21 mole, 1.2 equivalents) and stirred under argon until the imidazole dissolved. The contents of the flask were cooled to 0°C and a solution of *tert*-butyldimethylsilyl chloride (676.6g, 4.49 mole, 1.28 equivalents) was added dropwise maintaining the temperature in the range ±3°C. The cooling bath was removed and the reaction mixture was allowed to warm to ambient temperature and stirred, under argon, for 20 hours. The reaction was analysed by TLC to confirm complete reaction then charged with saturated sodium hydrogen carbonate solution (51) and stirred for 20 minutes. The mixture was transferred to a separator and the layers were allowed to separate. The aqueous phase was extracted with dichloromethane (21) and the combined organic phases were washed with saturated brine solution (21). The organic phase was dried over magnesium sulfate (700g) then filtered and the filtrate was evaporated, under reduced pressure, to give product (2) as a brown oil weighing 1602.6g (108%), sufficiently pure for use in the next step.

The spectroscopic data obtained were fully consistent with structure (2).

### Example 2 (Reaction scheme Step 2)

### (±)-2-(2-allyloxy-3-bromophenyl)-4-(tert-butyldimethylsilanyloxo)-3-(2-methylpropenyl)-cyclopentanone (3).

A suspension of CuBr.DMS (185.5g, 0.90 mole, 1.5 equivalents) was prepared under dry argon and cooled to -30°C with stirring. A tetrahydrofuran solution of 2-methyl-1-propenyl magnesium bromide (3.241 at 0.5M, 1.62 mole, 2.7 equivalents) was added, by cannula, over 45 minutes maintaining the temperature of reaction at -30°C. The reaction mixture was stirred at -30°C for a further 45 minutes then a solution of (2) (254g, 0.6 mole, 1 equivalent) in dry tetrahydrofuran (11) mixed with chlorotrimethylsilane (510ml, 4 mole, 6.7 equivalents) was added by cannula keeping the reaction temperature between -30°C and -35°C. After stirring at this temperature for 3 hours (until the reaction was complete by TLC analysis), the reaction mixture was cooled to -40°C and quenched with portions of saturated ammonium chloride solution (total 21), initially holding the temperature below -25°C. The mixture was transferred to a separator with water (2.51) and *tert*-butyl methyl ether (1.61) rinses. The aqueous phase was separated and extracted twice with *tert*-butyl methyl ether (total 3.21) then the combined organic phases were washed with saturated sodium hydrogen carbonate solution (1 x 1.61, 5 x 11). The organic phase was washed with saturated brine solution (1.61) and dried with magnesium sulfate (250g). Filtration and evaporation under reduced pressure at 20°C gave crude product as a brown oil (299.1g).

The crude product was dissolved in heptane fraction (0.51) and purified by filtration through a pad of silica (2kg) eluting with heptane fraction (51) and then 15:1 heptane fraction: ethyl acetate (201). Fractions containing product (on the basis of TLC) were combined and evaporated, under reduced pressure, to give product (3) as a yellow oil (187.4g, 65%).

The spectroscopic data obtained were fully consistent with structure (3).

### Example 3 (Reaction scheme Step 3)

### (±)-2-Bromo-6-[3-(tert-butyldimethylsilanyloxy)-5-hydroxy-2-(2-methylpropenyl)-cyclopentyl]-phenol (4).

A solution of (3) (634.7g, 1.32mole, 1 equivalent) was prepared in methanol (5.51) in a dry flask, under argon. Pd(PPh₃)₄ (15.3g, 0.132mole, 1 mole%) was charged to the yellow solution and the equipment was shielded from light. The reaction mixture was cooled to -2°C to -5°C and small portions of NaBH₄ (total 62.4g, 1.65 mole, 1.25 equivalents) were added over 55 minutes, under anhydrous conditions, keeping the reaction temperature in the range 10°C to 20°C. After a further 15 minutes, reaction completion was confirmed by TLC analysis and the reaction mixture was cooled whilst the reaction was quenched with saturated brine solution (4.51). The mixture was stirred for 10 minutes without cooling, then charged with *tert*-butyl methyl ether (6.61) and stirred for 10 minutes and allowed to settle for 10 minutes. The supernatant was transferred to a separator and the layers were separated running the aqueous layer into the reaction flask and adding water (1.51) to dissolve the solids. The aqueous layer was extracted with *tert*-butyl methyl ether(1 x 31, 1 x 21), retaining any interfacial material with the aqueous phase. The combined organic extracts were washed with saturated brine solution (2 x 31) then dried with magnesium sulfate (500g). Filtration and evaporation, under reduced pressure, gave product as a dark golden oil (555.2g, 95%).

The spectroscopic data obtained were fully consistent with structure (4).

### Example 4 (Reaction scheme Step 4)

### (±)-[7-Bromo-3-(2-methylpropenyl)-2,3,3a,8a-tetrahydro-1H-8-oxa-cyclopenta[a]inden-2-yloxy]-tert-butyldimethylsilane (5).

A solution of (4) (425.6g, 0.964mole, 1 equivalent) was prepared in anhydrous tetrahydrofuran (3.91) in a dry flask, under argon. Triphenylphosphine (256g, 0.97mole, 1.01 equivalent) was charged then the solution was chilled to -30°C and diisopropylazodicarboxylate (237 ml, 1.20 mole, 1.25 equivalents) was rapidly dripped in over 30 minutes at a temperature of -30°C completing the addition with an anhydrous tetrahydrofuran rinse (50ml). Reaction completion was confirmed by TLC analysis then water (50ml) was added to the reaction mixture which was stirred for 5 minutes. Solvent was removed by evaporation under reduced pressure and the residue was dissolved in *tert*-butyl methyl ether (21) and transferred to a separator with a water rinse (2.21). The mixture was stirred for 5 minutes then allowed to separate and the aqueous layer run off to waste. The organic phase was diluted with *tert*-butyl methyl ether (21) and the resulting solution washed sequentially with water (2.21) and saturated brine (2.21). The organic phase was dried with magnesium sulfate (420g). After filtration, the filtrate was evaporated to dryness under reduced pressure. The resulting solid was extracted with heptane fraction (1.51) and the slurry was filtered under suction. The solids were further washed with heptane fraction (3 x 1.51) and the filtrate was evaporated to give crude product as an orange oil.

The crude product was dissolved in heptane fraction (250ml) and purified by filtration through a silica pad (3kg) rinsing through with 50:1 heptane fraction:ethyl acetate (5 x 21) and 25:1 heptane fraction:ethyl acetate (7 x 2.61) collecting 21 then 2.61 fractions. Fractions containing product, by TLC analysis, were combined and evaporated, under reduced pressure to give product (5) as a yellow oil weighing 367g (90%).

The spectroscopic data obtained were fully consistent with structure (5).

### Example 5 (Reaction scheme Step 5)

### (±)-4-[2-(tert-Butyldimethylsilanyloxy)-3-(2-methylpropenyl)-2,3,3a,8a-tetrahydro-1H-8-oxa-cyclopenta[a]inden-7-yl]-butyric acid methyl ester (6).

A dry flask equipped with stirrer, argon bubbler, temperature probe, septum cap and stopper was charged with 9-BBN (9-borabicyclo[3.3.1]nonane) in THF (2.481 at 0.5M, 1.24 mole, 1.5 equivalents) by cannula. The solution was chilled to 10°C and a pre-dried solution of methyl 3-butenoate (124.1g, 1.24 mole, 1.5 equivalents) in anhydrous tetrahydrofuran (125ml) was added, by cannula, rinsing in with anhydrous tetrahydrofuran (2 x 50ml). The reaction mixture was allowed to warm to ambient temperature and stirred for 2 hours then complete formation of the intermediate was confirmed by TLC analysis. Triphenylarsine (25.3g, 0.0826 mole, 0.1 equivalents) followed by PdCl₂(dppf)₂ (20.25g, 0.0248 mole, 0.03 equivalents) were added under anhydrous conditions. A pre-dried solution of (5) (350g, 0.826 mole, 1 equivalent) in anhydrous tetrahydrofuran (350ml) was charged to the reaction flask by cannula, rinsing in with anhydrous tetrahydrofuran (2 x 50ml). Sodium methoxide (66.9g, 1.238 mole, 1.5 equivalents) was added, under anhydrous conditions, and the septum cap was replaced with a condenser set for reflux with argon bubbler. The stirred reaction mixture was heated at 50°C to 60°C for 16 hours. Reaction completion was confirmed by TLC analysis and the reaction mixture was cooled to 10°C and charged with *tert*-butyl methyl ether (2.51) and saturated brine solution (2.81) and the resulting mixture was transferred to a separator rinsing through with *tert*-butyl methyl ether (0.31). The mixture was stirred and allowed to separate and the aqueous phase was further extracted with *tert*-butyl methyl ether (0.71). The combined organic phases were washed with saturated brine (0.71) and dried with magnesium sulfate (300g). The mixture was filtered and the filtrate was evaporated, under reduced pressure, to give a brown oil (containing solids) which was chilled to 0°C to 5°C and treated with heptane fraction (1.41) with stirring. The mixture was filtered through celite (70g), rinsing through with portions of heptane fraction (4 x 350ml) and the resulting filtrate was evaporated, under reduced pressure, to give an orange oil. This material was further purified by passage through a pad of silica (2.4kg) using gradient elution with heptane fraction (101), 4:1 heptane fraction:dichloromethane (61), 3:2 heptane fraction:dichloromethane (61) and 9:1 heptane fraction:ethyl acetate (61) as eluent. Repeated purification through silica gave (6) (total 282.6g, 76.9%) as a yellow oil.

The spectroscopic data obtained were fully consistent with structure (6).

### Example 6 (Reaction scheme Step 6)

### (±)-4-[2-(tert-Butyldimethylsilanyloxy)-3-(1,2-dihydroxy-2-methylpropyl)-2,3,3a,8a-tetrahydro-1H-8-oxa-cyclopenta[a]inden-7-yl]-butyric acid methyl ester (7).

A reaction flask was charged with compound (6) (294.9g, 0.663 mole, 1 equivalent) dissolved in tetrahydrofuran (1.771). Water (295ml) and N-methylmorpholine N-oxide (140g, 1.19 mole, 1.8 equivalents) were added, under argon, and the stirred mixture was cooled to -5°C to -10°C. Aqueous 4% osmium tetroxide solution (60ml, 0.008 mole, 0.14 equivalents) was added and the reaction mixture was stirred under argon at ambient temperature for 62 hours (until TLC analysis showed no starting material remaining). The flask was charged with solid sodium hydrosulfite (58.9g) and stirred for 15 minutes. The mixture was allowed to stand for 30 minutes then the supematent was decanted and filtered through celite (20g). The residue was diluted with ethyl acetate (500ml) and filtered through the celite pad to give a biphasic filtrate. The filtrate was evaporated, under reduced pressure, to remove solvent and the residue was partitioned with water (11) and *tert*-butyl methyl ether (11). The organic phase was washed with 10% potassium hydrogen sulfate solution (450ml) and four times with saturated brine solution (1x 250ml, 1x 100ml, 2 x 50ml). The organic phase was dried with magnesium sulfate (100g), filtered and evaporated under reduced pressure to give crude product as a reddish-brown oil (345.9g). The crude product was allowed to stand at 4°C overnight then heptane fraction (500ml) was added to the partially crystalline material and the mixture was allowed to stand for a further 1 hour at 4°C. Solids were filtered off under suction and washed with heptane fraction (2 x 100ml) to give after vacuum drying 70.8g off-white solid (solid diastereomer). The filtrate was evaporated and purified by chromatography on silica (1.5kg) using gradient elution [heptane fraction (41), 4:1 heptane fraction:ethyl acetate (41), 3:1 heptane fraction:ethyl acetate (41), 2:1 heptane fraction:ethyl acetate (41), 1:1 heptane fraction:ethyl acetate (41), ethyl acetate (21), ethyl acetate containing 1% methanol (21), ethyl acetate containing 2.5% methanol, ethyl acetate containing 5% methanol, ethyl acetate containing 10% methanol]. Repeated purification on silica gave a total of 199.6g of oily diastereomer. Total yield of (7), as solid plus oil, was 270.4g (85.2%).

The spectroscopic data obtained were fully consistent with structure (7).

### Example 7 (Reaction scheme Step 7)

### (±)-4-[2-(tert-Butyldimethylsilanyloxy)-3-formyl-2,3,3a,8a-tetrahydro-1H-8-oxa-cyclopenta[a]inden-7-yl]-butyric acid methyl ester (8).

A reaction flask was charged with (7) (259.5g, 0.542 mole, 1 equivalent) and tetrahydrofuran (2.61). The flask was stirred, under argon, and charged with water (0.651) and sodium periodate (231.9g, 1.084 mole, 2 equivalents). The reaction mixture was stirred at ambient temperature for 75 minutes then reaction completion confirmed by TLC analysis. The reaction mixture was partitioned between heptane fraction (11) and saturated brine solution (11) and the aqueous phase was extracted three times with heptane fraction (0.51, 0.41, 0.251) and the combined organic phases were washed twice with saturated brine solution (0.751, 0.51). The organic phase was dried over magnesium sulfate (150g) then filtered and evaporated under reduced pressure at a bath temperature not exceeding 25°C to give (8) (231.8g, 101.9%) as a colourless oil (containing a trace of solvent).

The spectroscopic data obtained were fully consistent with structure (8).

## Claims

1. A method for the preparation of an intermediate for the synthesis of a benzoprostacyclin comprising at least one of the following seven steps:
Step 1: the protection of (±)-2-(2-allyloxy-3-bromophenyl)-4-hydroxy-cyclopent-2-enone (1) with *tert*-butyldimethylsilyl chloride to give (±)-2-(2-allyloxy-3-bromophenyl)-4-(*tert*-butyldimethylsilanyloxo)-cyclopent-2-enone (2);
Step 2: the reaction of (±)-2-(2-allyloxy-3-bromophenyl)-4-(*tert-*butyldimethylsilanyloxo)-cyclopent-2-enone (2) with 2-methyl-1-propenyl magnesium bromide via a copper mediated 1,4 addition to give (±)-2-(2-allyloxy-3-bromophenyl)-4-(*tert*-butyldimethylsilanyloxo)-3-(2-methylpropenyl)-cyclopentanone (3);
Step 3: the reaction of (±)-2-(2-allyloxy-3-bromophenyl)-4-(*tert-*butyldimethylsilanyloxo)-3-(2-methylpropenyl)-cyclopentanone (3) with a hydride reducing agent in the presence of a palladium catalyst to give (±)-2-bromo-6-[3-(*tert-*butyldimethylsilanyloxy)-5-hydroxy-2-(2-methylpropenyl)-cyclopentyl]-phenol (4);
Step 4: the reaction of (±)-2-bromo-6-[3-(*tert*-butyldimethylsilanyloxy)-5-hydroxy-2-(2-methylpropenyl)-cyclopentyl]-phenol (4) under Mitsonobu conditions to give (±)-[7-bromo-3-(2-methylpropenyl)-2,3,3a,8a-tetrahydro-1H-8-oxa-cyclopenta[a]inden-2-yloxy]-*tert*-butyldimethylsilane (5);
Step 5: the reaction of (±)-[7-bromo-3-(2-methylpropenyl)-2,3,3a,8a-tetrahydro-1H-8-oxa-cyclopenta[a]inden-2-yloxy]-*tert*-butyldimethylsilane (5) with a methyl 3-butenoate/borane adduct to give (±)-4-[2-(*tert*-butyldimethylsilanyloxy)-3-(2-methylpropenyl)-2,3,3a,8a-tetrahydro-1H-8-oxa-cyclopenta[a]inden-7-yl]-butyric acid methyl ester (6);
Step 6: the reaction of (±)-4-[2-(*tert*-butyldimethylsilanyloxy)-3-(2- methylpropenyl)-2,3,3a,8a-tetrahydro-1H-8-oxa-cyclopenta[a]inden-7-yl]-butyric acid methyl ester (6) with osmium tetroxide and a re-oxidant to give (±)-4-[2-(*tert*-butyldimethylsilanyloxy)-3-(1,2-dihydroxy-2-methylpropyl)-2,3,3a,8a-tetrahydro-1H-8-oxa-cyclopenta[a]inden-7-yl]-butyric acid methyl ester (7);
Step 7: the reaction of (±)-4-[2-(*tert*-butyldimethylsilanyloxy)-3-(1,2-dihydroxy-2-methylpropyl)-2,3,3a,8a-tetrahydro-1H-8-oxa-cyclopenta[a]inden-7-yl]-butyric acid methyl ester (7) with sodium periodate to give (±)-4-[2-(*tert*-butyldimethylsilanyloxy)-3-formyl-2,3,3a,8a-tetrahydro-1H-8-oxa-cyclopenta[a]inden-7-yl]-butyric acid methyl ester (8).

2. A method according to claim 1, comprising the preparation of (±)-2-(2-allyloxy-3-bromophenyl)-4-(*tert*-butyldimethylsilanyloxo)-3-(2-methylpropenyl)-cyclopentanone (3) according to step 2.

3. A method according to claim 2, wherein (±)-2-(2-allyloxy-3-bromophenyl)-4-(*tert*-butyldimethylsilanyloxo)-cyclopent-2-enone (2) is reacted with 2-methyl-1-propenyl bromide in the presence of a CuBr.DMS complex and chlorotrimethylsilane.

4. A method according to claim 1, comprising the preparation (±)-2-bromo-6-[3-(*tert*-butyldimethylsilanyloxy)-5-hydroxy-2-(2-methylpropenyl)-cyclopentyl]-phenol (4) according to step 3.

5. A method according to claim 4, wherein (±)-2-(2-allyloxy-3-bromophenyl)-4-(*tert*-butyldimethylsilanyloxo)-3-(2-methylpropenyl)-cyclopentanone (3) is reacted with sodium borohydride in the presence of Pd(Ph₃)₄.

6. A method according to claim 1, comprising the preparation of (±)-[7-promo-3-(2-methylpropenyl)-2,3,3a,8a-tetrahydro-1H-8-oxa-cyclopenta[a]inden-2-yloxy]-*tert-*butyldimethylsilane (5) according to step 4.

7. A method according to claim 6, wherein (±)-2-bromo-6-[3-(*tert-*butyldimethylsilanyloxy)-5-hydroxy-2-(2-methylpropenyl)-cyclopentyl]-phenol (4) is reacted with triphenylphosphine and diisopropylazodicarboxylate.

8. A method according to claim 1, comprising the preparation of (±)-4-[2-(*tert-*butyldimethylsilanyloxy)-3-(2-methylpropenyl)-2,3,3a,8a-tetrahydro-1H-8-oxa-cyclopenta[a]inden-7-yl]-butyric acid methyl ester (6) according to step 5.

9. A method according to claim 8, wherein (±)-[7-bromo-3-(2-methylpropenyl)-2,3,3a,8a-tetrahydro-1H-8-oxa-cyclopenta[a]inden-2-yloxy]-*tert*-butyldimethylsilane (5) is reacted with methyl 3-butenoate and 9-BBN in the presence of PdCl₂(dppf)₂ and triphenylarsine.

10. A method according to claim 1, comprising the preparation of (±)-4-[2-(*tert-*butyldimethylsilanyloxy)-3-(1,2-dihydroxy-2-methylpropyl)-2,3,3a,8a-tetrahydro-1H-8-oxa-cyclopenta[a]inden-7-yl]-butyric acid methyl ester (7) according to step 6.

11. A method according to claim 10, wherein (±)-4-[2-(*tert-*butyldimethylsilanyloxy)-3-(2-methylpropenyl)-2,3,3a,8a-tetrahydro-1H-8-oxa-cyclopenta[a]inden-7-yl]-butyric acid methyl ester (6) is reacted with osmium tetroxide and N-methylmorpholine N-oxide.

12. A method according to any preceding claim, including the preparation of (±)-4-[2-(*tert*-butyldimethylsilanyloxy)-3-formyl-2,3,3a,8a-tetrahydro-1H-8-oxa-cyclopenta[a]inden-7-yl]-butyric acid methyl ester (8).

13. A method for the preparation of Beraprost comprising a method as claimed in any preceding claim, for the synthesis of an intermediate followed by the synthesis of Beraprost from the intermediate so formed.

14. A compound (±)-2-(2-allyloxy-3-bromophenyl)-4-(*tert-*butyldimethylsilanyloxo)-3-(2-methylpropenyl)-cyclopentanone (3).

15. A compound (±)-2-bromo-6-[3-(*tert*-butyldimethylsilanyloxy)-5-hydroxy-2-(2-methylpropenyl)-cyclopentyl]-phenol (4).

16. A compound (±)-[7-bromo-3-(2-methylpropenyl)-2,3,3a,8a-tetrahydro-1H-8-oxa-cyclopenta[a]inden-2-yloxy]-*tert*-butyldimethylsilane (5).

17. A compound (±)-4-[2-(*tert*-butyldimethylsilanyloxy)-3-(2-methylpropenyl)-2,3,3a,8a-tetrahydro-1H-8-oxa-cyclopenta[a]inden-7-yl]-butyric acid methyl ester (6).

18. A compound (±)-4-[2-(*tert*-butyldimethylsilanyloxy)-3-(1,2-dihydroxy-2-methylpropyl)-2,3,3a,8a-tetrahydro-1H-8-oxa-cyclopenta[a]inden-7-yl]-butyric acid methyl ester (7).

19. The compound (±)-4-[2-(*tert*-butyldimethylsilanyloxy)-3-formyl-2,3,3a,8a-tetrahydro-1H-8-oxa-cyclopenta[a]inden-7-yl]-butyric acid methyl ester (8).

## Patentansprüche

1. Verfahren zur Herstellung einer Zwischenverbindung zur Synthese eines Benzoprostacyclins, wobei das Verfahren mindestens einen der folgenden sieben Schritte umfasst:
Schritt 1: das Schützen von (±)-2-(2-Allyloxy-3-bromphenyl)-4-hydroxycyclopent-2-enon (1) mit *tert*.-Butyldimethylsilylchlorid, um (±)-2-(2-Allyloxy-3-bromphenyl)-4-(*tert*.-butyldimethylsilanyloxo)cyclopent-2-enon (2) zu erhalten;
Schritt 2: das Umsetzen von (±)-2-(2-Allyloxy-3-bromphenyl)-4-(*tert*.-butyldimethylsilanyloxo)cyclopent-2-enon (2) mit 2-Methyl-1-propenylmagnesiumbromid mittels einer kupfervermittelten 1,4-Addition, um (±)-2-(2-Allyloxy-3-bromphenyl)-4-(*tert*.-butyldimethylsilanyloxo)-3-(2-methylpropenyl)cyclopentanon (3) zu erhalten;
Schritt 3: das Umsetzen von (±)-2-(2-Allyloxy-3-bromphenyl)-4-(*tert*.-butyldimethylsilanyloxo)-3-(2-methylpropenyl)cyclopentanon (3) mit einem Hydridreduzierungsmittel in Gegenwart eines Palladiumkatalysators, um (±)-2-Brom-6-[3-(*tert*.-butyldimethylsilanyloxy)-5-hydroxy-2-(2-methylpropenyl)cyclopentyl]phenol (4) zu erhalten;
Schritt 4: das Umsetzen von (±)-2-Brom-6-[3-(*tert*.-butyldimethylsilanyloxy)-5-hydroxy-2-(2-methylpropenyl)cyclopentyl]phenol (4) unter Mitsonobu-Bedingungen, um (±)-[7-Brom-3-(2-methylpropenyl)-2,3,3a,8a-tetrahydro-1H-8-oxa-cyclopenta[a]inden-2-yloxy]-*tert*.-butyldimethylsilan (5) zu erhalten;
Schritt 5: das Umsetzen von (±)-[7-Brom-3-(2-methylpropenyl)-2,3,3a,8a-tetrahydro-1H-8-oxa-cyclopenta[a]inden-2-yloxy]-*tert*.-butyldimethylsilan (5) mit einem Methyl-3-butenoat/-boran-Addukt, um (±)-4-[2-(*tert*.-Butyldimethylsilanyloxy)-3-(2-methylpropenyl)-2,3,3a,8a-tetrahydro-1H-8-oxacyclopenta[a]inden-7-yl]buttersäuremethylester (6) zu erhalten;
Schritt 6: das Umsetzen von (±)-4-[2-(*tert*.-Butyldimethylsilanyloxy)-3-(2-methylpropenyl)-2,3,3a,8a-tetrahydro-1H-8-oxacyclopenta[a]inden-7-yl]buttersäuremethylester (6) mit Osmiumtetroxid und einem Reoxidans, um (±)-4-[2-(*tert*.-Butyldimethylsilanyloxy)-3-(1,2-dihydroxy-2-methylpropyl)-2,3,3a,8a-tetrahydro-1H-8-oxacyclopenta[a]inden-7-yl]buttersäuremethylester (7) zu erhalten;
Schritt 7: das Umsetzen von (±)-4-[2-(*tert*.-Butyldimethylsilanyloxy)-3-(1,2-dihydroxy-2-methylpropyl)-2,3,3a,8a-tetrahydro-1H-8-oxacyclopenta[a]inden-7-yl]buttersäuremethylester (7) mit Natriumperiodat, um (±)-4-[2-(*tert*.-Butyldimethylsilanyloxy)-3-formyl-2,3,3a,8a-tetrahydro-1H-8-oxacyclopenta[a]inden-7-yl]buttersäuremethylester (8) zu erhalten.

2. Verfahren nach Anspruch 1, das die Herstellung von (±)-2-(2-Allyloxy-3-bromphenyl)-4-(*tert*.-butyldimethylsilanyloxo)-3-(2-methylpropenyl)cyclopentanon (3) gemäß Schritt 2 umfasst.

3. Verfahren nach Anspruch 2, wobei (±)-(2-Allyloxy-3-bromphenyl)-4-(*tert*.-butyldimethylsilanyloxo)cyclopent-2-enon (2) mit 2-Methyl-1-propenylbromid in Gegenwart eines CuBr.DMS-Komplexes und Chlortrimethylsilan umgesetzt wird.

4. Verfahren nach Anspruch 1, das die Herstellung von (±)-2-Brom-6-[3-(*tert*.-butyldimethylsilanyloxy)-5-hydroxy-2-(2-methylpropenyl)cyclopentyl]phenol (4) gemäß Schritt 3 umfasst.

5. Verfahren nach Anspruch 4, wobei (±)-2-(2-Allyloxy-3-bromphenyl)-4-(*tert*.-butyldimethylsilanyloxo)-3-(2-methylpropenyl)cyclopentanon (3) mit Natriumborhydrid in Gegenwart von Pd(Ph₃)₄ umgesetzt wird.

6. Verfahren nach Anspruch 1, das die Herstellung von (±)-[7-Brom-3-(2-methylpropenyl)-2,3,3a,8a-tetrahydro-1H-8-oxa-cyclopenta[a]inden-2-yloxy]-*tert*.-butyldimethylsilan (5) gemäß Schritt 4 umfasst.

7. Verfahren nach Anspruch 6, wobei (±)-2-Brom-6-[3-(*tert*.-butyldimethylsilanyloxy)-5-hydroxy-2-(2-methylpropenyl)cyclopentyl]phenol (4) mit Triphenylphosphin und Diisopropylazodicarboxylat umgesetzt wird.

8. Verfahren nach Anspruch 1, das die Herstellung von (±)-4-[2-(*tert*.-Butyldimethylsilanyloxy)-3-(2-methylpropenyl)-2,3,3a,8a-tetrahydro-1H-8-oxacyclopenta[a]inden-7-yl]buttersäuremethylester (6) gemäß Schritt 5 umfasst.

9. Verfahren nach Anspruch 8, wobei (±)-[7-Brom-3-(2-methylpropenyl)-2,3,3a,8a-tetrahydro-1H-8-oxa-cyclopenta[a]inden-2-yloxy]-*tert*.-butyldimethylsilan (5) mit Methyl-3-butenoat und 9-BBN in Gegenwart von PdCl₂(dppf)₂ und Triphenylarsin umgesetzt wird.

10. Verfahren nach Anspruch 1, das die Herstellung von (±)-4-[2-(*tert*.-Butyldimethylsilanyloxy)-3-(1,2-dihydroxy-2-methylpropyl)-2,3,3a,8a-tetrahydro-1H-8-oxacyclopenta[a]inden-7-yl]buttersäuremethylester (7) gemäß Schritt 6 umfasst.

11. Verfahren nach Anspruch 10, wobei (±)-4-[2-(*tert*.-Butyldimethylsilanyloxy)-3-(2-methylpropenyl)-2,3,3a,8a-tetrahydro-1H-8-oxacyclopenta[a]inden-7-yl]buttersäuremethylester (6) mit Osmiumtetroxid und N-Methylmorpholin-N-oxid umgesetzt wird.

12. Verfahren nach einem der vorhergehenden Ansprüche, das die Herstellung von (±)-4-[2-(*tert*.-Butyldimethylsilanyloxy)-3-formyl-2,3,3a,8a-tetrahydro-1H-8-oxacyclopenta[a]inden-7-yl]buttersäuremethylester (8) beinhaltet.

13. Verfahren zur Herstellung von Beraprost, das ein Verfahren nach einem der vorhergehenden Ansprüche umfasst, zur Synthese einer Zwischenverbindung, gefolgt von der Synthese von Beraprost aus der so gebildeten Zwischenverbindung.

14. Verbindung (±)-2-(2-Allyloxy-3-bromphenyl)-4-(*tert*.-butyldimethylsilanyloxo)-3-(2-methylpropenyl)cyclopentanon (3).

15. Verbindung (±)-2-Brom-6-[3-(*tert*.-butyldimethylsilanyloxy)-5-hydroxy-2-(2-methylpropenyl)cyclopentyl]phenol (4).

16. Verbindung (±)-[7-Brom-3-(2-methylpropenyl)-2,3,3a,8a-tetrahydro-1H-8-oxa-cyclopenta[a]inden-2-yloxy]-*tert*.-butyldimethylsilan (5).

17. Verbindung (±)-4-[2-(*tert*.-Butyldimethylsilanyloxy)-3-(2-methylpropenyl)-2,3,3a,8a-tetrahydro-1H-8-oxacyclopenta[a]inden-7-yl]buttersäuremethylester (6).

18. Verbindung (±)-4-[2-(*tert*.-Butyldimethylsilanyloxy)-3-(1,2-dihydroxy-2-methylpropyl)-2,3,3a,8a-tetrahydro-1H-8-oxacyclopenta[a]inden-7-yl]buttersäuremethylester (7).

19. Verbindung (±)-4-[2-(*tert*.-Butyldimethylsilanyloxy)-3-formyl-2,3,3a,8a-tetrahydro-1H-8-oxacyclopenta[a]inden-7-yl]buttersäuremethylester (8).

## Revendications

1. Procédé pour la préparation d'un produit intermédiaire pour la synthèse d'une benzoprostacycline comprenant au moins une des sept étapes suivantes :
Étape 1 : la protection du chlorure de (±)-2-(2-allyloxy-3-bromophényl)-4-hydroxy-cyclopent-2-énone (1) avec du chlorure de *tert*-butyldiméthylsilyle pour obtenir la (±)-2-(2-allyloxy-3-bromophényl)-4-(*tert*-butyldiméthylsilanyloxo)-cyclopent-2-énone (2) ;
Étape 2 : la mise en réaction de la (±)-2-(2-allyloxy-3-bromophényl)-4-(*tert-*butyldiméthylsilanyloxo)-cyclopent-2-énone (2) avec du bromure de 2-méthyl-1-propényl magnésium via une addition 1,4 dans laquelle intervient le cuivre comme médiateur pour obtenir la (±)-2-(2-allyloxy-3-bromophényl)-4-(*tert-*butyldiméthylsilanyloxo)-3-(2-méthylpropényl)-cyclopentanone (3) ;
Étape 3 : la mise en réaction de la (±)-2-(2-allyloxy-3-bromophényl)-4-(*tert-*butyldiméthylsilanyloxo)-3-(2-méthylpropényl)-cyclopentanone (3) avec un hydrure à titre d'agent de réduction en présence d'un catalyseur à base de palladium pour obtenir le (±)-2-bromo-6-[3-(*tert*-butyldiméthylsilanyloxy)-5-hydroxy-2-(2-méthylpropényl)-cyclopentyl]-phénol (4) ;
Étape 4 : la mise en réaction du (±)-2-bromo-6-[3-(*tert*-butyldiméthylsilanyloxy)-5-hydroxy-2-(2-méthylpropényl)-cyclopentyl]-phénol (4) dans des conditions de Mitsonobu pour obtenir le (±)-[7-bromo-3-(2-méthylpropényl)-2,3,3a,8a-tétrahydro-1H-8-oxa-cyclopenta[a]indén-2-yloxy]-*tert*-butyldiméthylsilane (5) ;
Étape 5 : la mise en réaction du (±)-[7-bromo-3-(2-méthylpropényl)-2,3,3a,8a-tétrahydro-1H-8-oxa-cyclopenta[a]indén-2-yloxy]-tert-butyldiméthylsilane (5) avec un adduit de 3-buténoate de méthyle/borane pour obtenir l'ester méthylique de l'acide (±)-4-[2-(*tert*-butyldiméthylsilanyloxy)-3-(2-méthylpropényl)-2,3,3a,8a-tétrahydro-1H-8-oxa-cyclopenta[a]indén-7-yl]-butyrique (6) ;
Étape 6 : la mise en réaction de l'ester méthylique de l'acide (±)-4-[2-(*tert-*butyldiméthylsilanyloxy)-3-(2-méthylpropényl)2,3,3a,8a-tétrahydro-1H-8-oxa-cyclopenta[a]indén-7-yl]-butyrique (6) avec du tétroxyde d'osmium et un réoxydant pour obtenir l'ester méthylique de l'acide (±)-4-[2-(*tert-*butyldiméthylsilanyloxy)-3-(1,2-dihydroxy-2-méthylpropyl)-2,3,3a,8a-tétrahydro-1H-8-oxa-cyclopenta[a]indén-7-yl]-butyrique (7) ;
Étape 7 : la mise en réaction de l'ester méthylique de l'acide (±)-4-[2-(*tert-*butyldiméthylsilanyloxy)-3-(1,2-dihydroxy-2-méthylpropyl)-2,3,3a,8a-tétrahydro-1H-8-oxa-cyclopenta[a]indén-7-yl]-butyrique (7) avec du periodate de sodium pour obtenir l'ester méthylique de l'acide (±)-4-[2-(*tert*-butyldiméthylsilanyloxy)-3-formyl-2,3,3a,8a-tétrahydro-1H-8-oxa-cyclopenta[a]indén-7-yl]-butyrique (8).

2. Procédé selon la revendication 1, comprenant la préparation de la (±)-2-(2-allyloxy-3-bromophényl)-4-(*tert*-butyldiméthylsilanyloxo)-3-(2-méthylpropényl)-cyclopentanone (3) conformément à l'étape 2.

3. Procédé selon la revendication 2, dans lequel on fait réagir de la (±)-2-(2-allyloxy-3-bromophényl)-4-(*tert*-butyldiméthylsilanyloxo)-cyclopent-2-énone (2) avec du bromure de 2-méthyl-1-propényle en présence d'un complexe CuBr.DMS et de chlorotriméthylsilane.

4. Procédé selon la revendication 1, comprenant la préparation du (±)-2-bromo-6-[3-(*tert*-butyldiméthylsilanyloxy)-5-hydroxy-2-(2-méthylpropényl)-cyclopentyl]-phénol (4) conformément à l'étape 3.

5. Procédé selon la revendication 4, dans lequel on fait réagir de la (±)-2-(2-allyloxy-3-bromophényl)-4-(*tert*-butyldiméthylsilanyloxo)-3-(2-méthylpropényl)-cyclopentanone (3) avec du borohydrure de sodium en présence de Pd(Ph₃)₄.

6. Procédé selon la revendication 1, comprenant la préparation du (±)-[7-bromo-3-(2-méthylpropényl)-2,3,3a,8a-tétrahydro-1H-8-oxa-cyclopenta[a]indén-2-yloxy]-*tert*-butyldiméthylsilane (5) conformément à l'étape 4.

7. Procédé selon la revendication 6, dans lequel on fait réagir du (±)-2-bromo-6-[3-(tert-butyldiméthylsilanyloxy)-5-hydroxy-2-(2-méthylpropényl)-cyclopentyl]-phénol (4) avec de la triphénylphosphine et du diisopropylazodicarboxylate.

8. Procédé selon la revendication 1, comprenant la préparation de l'ester méthylique de l'acide (±)-4-[2-(*tert*-butyldiméthylsilanyloxy)-3-(2-méthylpropényl)-2,3,3a,8a-tétrahydro-1H-8-oxacyclopenta[a]indén-7-yl]-butyrique (6) conformément à l'étape 5.

9. Procédé selon la revendication 8, dans lequel on fait réagir du (±)-[7-bromo-3-(2-méthylpropényl)-2,3,3a,8a-tétrahydro-1H-8-oxa-cyclopenta[a]indén-2-yloxy]-*tert*-butyldiméthylsilane (5) avec du 3-buténoate de méthyle et du 9-BBN en présence de PdCl₂(dppf)₂ et de triphénylarsine.

10. Procédé selon la revendication 1, comprenant la préparation de l'ester méthylique de l'acide (±)-4-[2-(*tert*-butyldiméthylsilanyloxy)-3-(1,2-dihydroxy-2-méthylpropyl)-2,3,3a,8a-tétrahydro-1H-8-oxa-cyclopenta[a]indén-7-yl]-butyrique (7) conformément à l'étape 6.

11. Procédé selon la revendication 10, dans lequel on fait réagir de l'ester méthylique de l'acide (±)-4-[2-(*tert*-butyldiméthylsilanyloxy)-3-(2-méthylpropényl)-2,3,3a,8a-tétrahydro-1H-8-oxacyclopenta[a]indén-7-yl]-butyrique (6) avec du tétroxyde d'osmium et du N-oxyde de N-méthylmorpholine.

12. Procédé selon l'une quelconque des revendications précédentes, englobant la préparation de l'ester méthylique de l'acide (±)-4-[2-(*tert-*butyldiméthylsilanyloxy)-3-formyl-2,3,3a,8a-tétrahydro-1H-8-oxacyclopenta-[a]indén-7-yl]-butyrique (8).

13. Procédé pour la préparation de Beraprost comprenant un procédé tel que revendiqué dans l'une quelconque des revendications précédentes, pour la synthèse d'un produit intermédiaire, suivie de la synthèse du Beraprost à partir du produit intermédiaire ainsi obtenu.

14. Composé (±)-2-(2-allyloxy-3-bromophényl)-4-(*tert*-butyldiméthylsilanyloxo)-3-(2-méthylpropényl)-cyclopentanone (3).

15. Composé (±)-2-bromo-6-[3-(*tert*-butyldiméthylsilanyloxy)-5-hydroxy-2-(2-méthylpropényl)-cyclopentyl]-phénol (4).

16. Composé (±)-[7-bromo-3-(2-méthylpropényl)-2,3,3a,8a-tétrahydro-1H-8-oxa-cyclopenta[a]indén-2-yloxy]-*tert*-butyldiméthylsilane (5).

17. Composé ester méthylique de l'acide (±)-4-[2-(tert-butyldiméthylsilanyloxy)-3-(2-méthylpropényl)-2,3,3a,8a-tétrahydro-1H-8-oxa-cyclopenta[a]indén-7-yl]-butyrique (6).

18. Composé ester méthylique de l'acide (±)-4-[2-(*tert-*butyldiméthylsilanyloxy)-3-(1,2-dihydroxy-2-méthylpropyl)-2,3,3a,8a-tétrahydro-1H-8-oxa-cyclopenta[a]indén-7-yl]-butyrique (7).

19. Composé ester méthylique de l'acide (±)-4-[2-(*tert-*butyldiméthylsilanyloxy)-3-formyl-2,3,3a,8a-tétrahydro-1H-8-oxa-cyclopenta-[a]indén-7-yl]-butyrique (8).
